# FASCICULE DE BREVET EUROPEEN

(11) **EP 4 312 674 B1**
(45) Date de publication et mention de la délivrance du brevet: **14.05.2025**
(21) Numéro de dépôt: 22715311.1
(22) Date de dépôt: 28.02.2022
(51) Int. Cl.: A47G 21/02, G09B 19/00, G16H 20/60, A47G 23/12, G16H 40/63

(54) **COUVERT INTELLIGENT ÉTANCHE RECHARGEABLE ET SON ÉTUI DE RECHARGEMENT**
WIEDERAUFLADBARES DICHTUNGSDICHTES INTELLIGENTES ESSBESTECK UND SEIN AUFLADEGEHÄUSE
RECHARGEABLE SEAL-TIGHT SMART CUTLERY AND ITS RECHARGING CASE

(30) Priorité: 01.03.2021 FR 2101948
(43) Date de publication de la demande: 07.02.2024
(73) Titulaire: SLOW CONTROL, 33260 La Teste de Buch (FR)
(72) Inventeur: LEPINE, Jacques, 95290 L'isle adam (FR); DEJOUX, Guillaume, 95290 L'isle adam (FR)
(86) Numéro de dépôt international: PCT/EP2022/054917
(87) Numéro de publication internationale: WO 2022/184607

(56) Documents cités:
- FR-A1- 3 000 371
- US-A1- 2010 240 962
- US-A1- 2014 347 491

## Description

La présente invention concerne un couvert intelligent étanche rechargeable et son étui de rechargement.

Par intelligent, il s'entend d'un dispositif électronique spécifique qui 1- aide à manger lentement en avertissant l'utilisateur s'il mange trop vite 2 - qui permet le rechargement.

Par couvert il s'entend d'une fourchette, d'une cuillère ou d'une baguette.

La présente invention propose une solution qui permet 1 - le lavage en machine du couvert intelligent 2 - un rechargement rapide de l'alimentation de son circuit électronique sans atteindre à l'étanchéité 3 - un prix de revient de fabrication optimisé.

Un couvert nécessite d'être lavé à chaque repas. La mise en fonction du caractère intelligent du couvert nécessite de recharger régulièrement la batterie qui alimente l'électronique qui pilote l'intelligence du couvert. Il n'existe pas dans l'art antérieur de solution de couvert intelligent qui soit étanche. Il existe des solutions de couverts intelligents à pile ou à rechargement par fil. Il existe notamment FR 2870096 A1 FR 2900809 A1 qui ne proposent pas de solution pour l'étanchéité. Le document FR 3 000 371 A1 divulgue le préambule de la revendication 1. Le souci de l'intégration d'une source d'alimentation externe pour réalimenter le couvert est qu'elle nécessite d'accéder fréquemment aux bornes du circuit intelligent pour pouvoir le recharger. Cet accès aux bornes du circuit rend l'étanchéité extrêmement compliquée donc trop couteuse pour pouvoir être intégrée à un couvert de table dans des conditions de coûts acceptables.

Les trois contraintes principales posées par un couvert de table intelligent rechargeable sont 1 - Tenir dans un volume mécanique réduit 2 - Etre lavé régulièrement 3 - Etre utilisé régulièrement et donc rechargé régulièrement.

Il est donc compris que pour résoudre ce problème, il est nécessaire de penser un circuit électronique optimisé qui s'agence particulièrement bien avec les éléments mécaniques. L'invention répond donc à une double contrainte électronique et mécanique pour réussir à atteindre son objectif.

Il est également compris que la mesure de la mise en bouche est une mesure qui est prise pendant le repas et qui avec la variété des utilisateurs et la variété des situations de conditions de repas est subtile, rapide et versatile. Cette mesure doit pouvoir être efficacement détectée et filtrée par le dispositif électronique. L'invention est définie par les caractéristiques de la revendication 1.

Dans un aspect principal, l'invention propose un circuit intelligent qui comprend un circuit électronique comprenant un circuit de charge, un circuit de détection, un circuit d'avertissement couplé à une super capacité logée dans un coffret étanche avec deux bornes en contact d'une part avec la tête de couvert et d'autre part avec le manche du couvert.

Dans un aspect de l'invention, les deux parties du couvert coopèrent chacune de deux façons avec le circuit électronique : Pour la tête de couvert 1 - être la sonde de détection de la mise en bouche 2 - être la borne de rechargement Plus du circuit. Pour le manche de couvert 1 - être la référence de détection du circuit 2 - être la borne de rechargement Moins du circuit de charge.

Il est donc compris que l'invention propose une sextuple coopération nouvelle entre le circuit électronique et les deux parties du couvert, à savoir : 1 - une coopération mécanique pour que l'ensemble assemblé ait une excellente tenue mécanique 2 - une coopération mécanique pour que les deux bornes du circuit soient en contact électrique avec les deux parties de couvert 3 - une coopération mécanique pour que l'ensemble constitue un ensemble étanche 4 - une coopération électronique pour que l'ensemble détecte les évènements de mise en bouche 5 - une coopération électronique pour le même ensemble se recharge à travers les mêmes bornes qui permettent la détection 6 - une double coopération mécanique -électronique de l'ensemble avec un boitier pour le rechargement du couvert.

L'invention sera mieux comprise à la lecture des figures annexées sur lesquelles :
[Fig 1] représente une vue des deux parties de couvert
[Fig 2] représente un vue de dessus de la carte électronique
[Fig 3] représente un vue de côté de la carte électronique
[Fig 4] représente une vue de côté de la carte électronique inscrite dans son boitier de protection
[Fig 5] représente l'assemblage de la carte électronique dans son boitier avec les deux parties de couvert
[Fig 6] représente les couverts dans leur étui de rechargement
[Fig 7] représente le schéma d'ensemble du circuit électronique intelligent
[Fig 8] représente le schéma qui précise le circuit de détection du circuit électronique intelligent

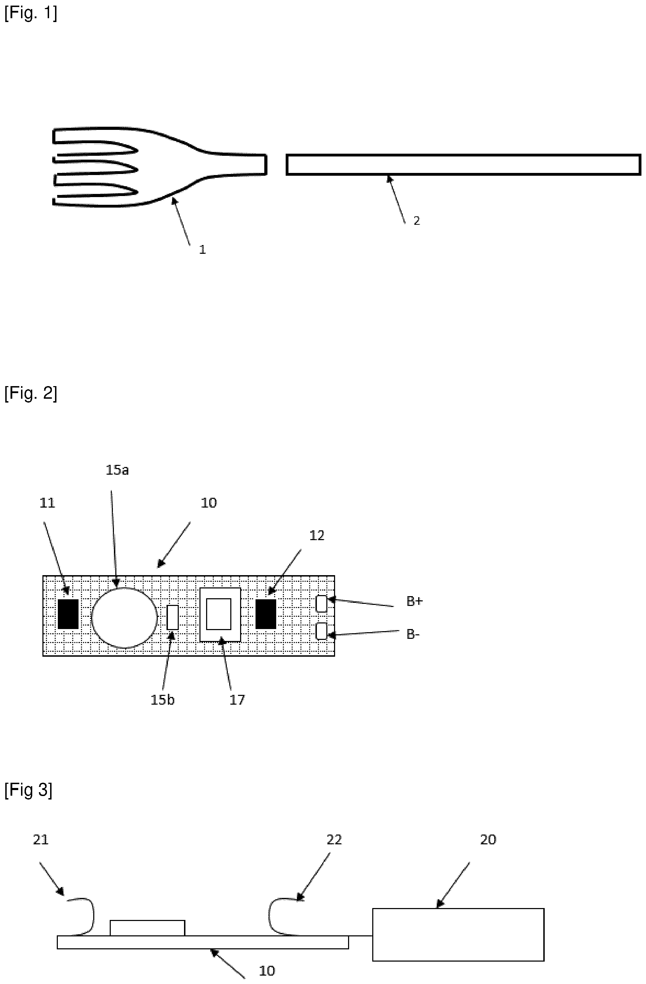

[Fig 1] représente une vue des deux parties de couvert avec une tête de couvert (1) qui comprend des dents et une épaule de raccordement au manche et un manche de couvert (2). Les deux parties de couverts raboutées reproduisent la forme générale d'un couvert. Elles sont séparées à proximité immédiate de l'épaule de la tête de couvert. Cette solution permet d'offrir une surface de préhension maximale du manche qui assurera un contact permanent avec la main du manche de couvert (2) lorsque l'utilisateur tiendra le couvert par le manche (2).

[Fig 2] représente un vue de dessus de la carte électronique (10). La carte électronique comprend un circuit dit de cœur (17) avec une puce et un circuit électrique dédié qui la relie aux éléments de charge et de détection ultérieurement décrit. Le circuit de cœur (17) est alimenté aux bornes (B+, B-) d'une alimentation. La carte comprend 1 - une plage de contact à la masse, dite plage de contact Moins (12) qui est reliée directement à la borne Moins (B-) du circuit 2 - une plage de contact dite Plus (11) qui est reliée à travers le circuit de cœur (17) à la borne (B+) 3 - des sorties d'avertissement (15) qui sont un vibreur (15a) et une lumière LED (15b) qui sont reliées au circuit de cœur (17). La fonction des soties d'avertissement (17) est de renseigner l'utilisateur par un signal d'avertissement vibratoire ou lumineux sur le fait qu'il mange trop vite ou pas.

[Fig 3] représente un vue de côté de la carte électronique (10), sur laquelle une super capacité (20) est branchée aux bornes d'alimentation (B+, B-) ; Comme chacun le sait une super capacité est une capacité ayant la possibilité de stocker une quantité importante de charges permettant ainsi de libérer du courant d'alimentation du circuit électronique. La nouveauté étant que les super capacités (20) n'alimentent pas jusqu'à aujourd'hui encore les couverts intelligents pour de nombreuses raisons qui sont notamment que les stockages de charges étaient insuffisants pour la demande en électricité nécessaire dans la conception des circuits électroniques tels que connus. Des progrès se faisant tant sur la capacité de charge par environnement volumique que de la conception du circuit électronique lui-même qui selon notre invention diminue radicalement la consommation, il devient possible avec la conjugaison de ces progrès de concevoir seulement maintenant une alimentation par super capacité pour peu que l'astuce majeure de recharger le circuit par les deux parties de couvert fût imaginé. En bref, l'intégration d'une super capacité au sein d'un couvert intelligent est rendu possible aux moyens conjugués de progrès technologiques sur la super capacité, de progrès technologiques sur le circuit électronique et de d'invention de l'intégration d'un circuit de chargement nouveau qui soit compatible avec ces progrès technologiques eux-mêmes. Sont apparents les deux ressorts de contacts, ressort de contact de borne Plus (21) et ressort de contact de borne Moins (22) qui font respectivement contacts en étant soudés aux plages de contact Plus (11) et plage de contact Moins (12).

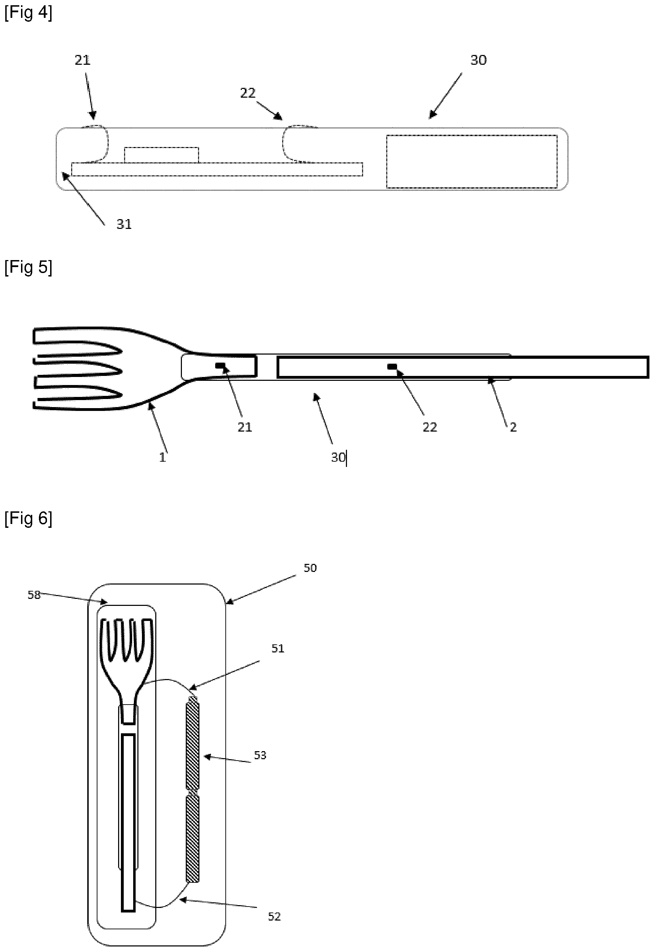

[Fig 4] représente une vue de côté de la carte électronique inscrite dans son boitier de protection (30). Le boitier forme une coque étanche de laquelle seuls les deux ressorts de contact (21, 22) sortent et affleurent. Une solution possible pour créer l'étanchéité au sein du boitier étant de remplir l'intérieur du boitier de résine isolante.

[Fig 5] représente l'assemblage de la carte électronique dans son boitier avec les deux parties de couvert (1, 2) avec le boitier (30) qui vient s'accrocher aux deux parties du couvert et créer la rigidité pour maintenir à l'ensemble la forme d'un couvert. Les moyens d'accroche spécifique d'accroche du boitier pour la tenue mécanique ne sont pas décrits dans ce brevet, mais la faisabilité est évidemment possible par de nombreux moyens qui sont soit le collage, soit la tenue mécanique au moyen de glissière d'accroches, soit la tenue mécanique au moyen de rivets.

[Fig 6] représente les couverts dans leur étui de rechargement (50) qui comprend une alimentation externe (53) en énergie électrique, ci-joint représenté par un jeu de deux piles dites bâton, mais qui aussi provenir d'un panneau solaire. La spécificité du boitier est qu'il comprend un circuit de calage (58) qui prend la forme du couvert et qui épouse le trajet des contacts de boitier (51, 52) qui viennent respectivement l'un faire contact depuis les bornes d'alimentation Plus et Moins de l'alimentation (53) respectivement en contact avec la tête de couvert (1) pour le contact de tête de boitier (51) et avec le manche (2) pour le contact de manche de boitier (52).

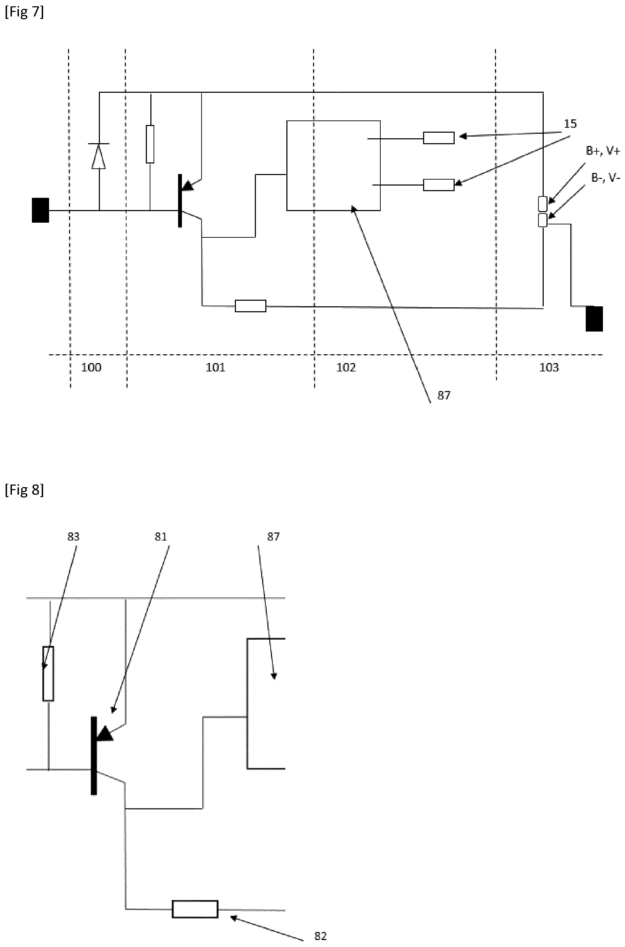

[Fig 7] représente le schéma d'ensemble du circuit électronique intelligent qui explique comment précisément la définition du circuit rend l'ensemble tel que décrit fonctionnel. La carte (10) loge de droite à gauche - Un circuit d'alimentation (103) avec la borne Plus (B+) au potentiel (V+), la borne (B-) au potentiel (V-) en l'occurrence à la masse - Un circuit d'avertissement (102) qui comprend le sorties d'avertissement (15), en l'occurrence le vibreur (15a) et la lumière LED (15b). Les sorties d'avertissement étant pilotées par une sortie sur la puce de calcul (87). - Un circuit de détection (101) ultérieurement décrit qui détecte les mises en bouche de la tête de couvert au moyen notamment d'un circuit de division de tension sur alimentation non régulée, par couplage de résistance de tirage, de résistance de masse et de transistor inversé. Ce circuit permet de détecter des grandes résistances et donc celle du corps qui relie la tête de fourchette au manche de fourchette lorsque l'utilisateur porte la fourchette à la bouche - Un circuit de charge (100) qui court-circuite astucieusement le circuit de détection (101), au cas où le circuit de charge soit alimenté par une source d'énergie. Le court-circuit étant en l'occurrence réalisé par une diode dite diode de charge dont la valeur de seuil est supérieure astucieusement calibrée à 0,7 V, c'est-à-dire qui fasse court-circuit sur cette branche du circuit si la personne est en train de manger et qui par contre laisse passer l'alimentation, typiquement au-dessus tension de la super capacité plus 0,7 V, et permette la charge de la super capacité, si le couvert est rangé dans son étui et soumis à une tension de charge.

[Fig 8] représente le schéma qui précise le circuit de détection du circuit électronique intelligent. Il faut voir que ce circuit ne répond à aucun autre circuit connu puisque sa contrainte est de détecter un signal de variation de tension très faible entre - une alimentation par une super capacité qui se vide et qui propose donc une tension qui n'est pas stable et qui décroit pendant l'utilisation - un microcontrôleur qui a des propriétés micro électrique propres qui peut perturber le circuit de détection - un circuit de charge qui ne doit pas perturber la mesure. Traditionnellement la solution qui serait proposée pour une détection de court-circuit par la mise en bouche du couvert serait d'utiliser un ampli opérationnel ou un comparateur. Le souci est que ces deux solutions nécessitent une tension d'alimentation stabilisée, ce qui n'est pas le cas dans le cas de l'alimentation par une super capacité. Clairement, au vu des éléments énoncés ci-dessus cette solution ne pourrait pas être fonctionnelle car l'ampli opérationnel interviendrait entre des bornes flottantes et ne réussirait pas donc à filtrer les événements. Le circuit doit donc ici conjuguer les contraintes du numérique et celles de l'analogique en détectant un évènement entre deux tensions variables. La solution proposée est d'utiliser une résistance dite de tirage (83) de grande valeur, c'est-à-dire en Méga ohm. Elle est branchée entre la base d'un transistor (81) et le V+ de la super capacité. Le transistor (81) est de type à polarité inversée dit PNP. La résistance de tirage tire le potentiel vers le haut pour éviter les fausses détections. L'ensemble de la résistance de tirage et de la résistance du corps humain constitue un diviseur de tension branché sur la base du transistor (81). En sortie de transistor il y une résistance dite de masse (82) de plusieurs centaines de kilos ohm évitant de perturber l'étage d'entrée du transistor. Sur le collecteur du transistor est branchée l'entrée de détection de la puce de calcul (87). L'ensemble est fonctionnel du fait de la calibration fine des résistances entourant le transistor. Ces calibrations permettant de détecter la présence d'une résistance de grande valeur comme celle du corps humain. En quelque sorte cette partie du circuit fonctionne comme un détecteur de très haute résistance en méga ohm mètre. L'utilisation d'un mégohmmètre étant impossible puisque dans ces appareils la tension d'alimentation est stable. Et enfin le fait de s'affranchir d'un régulateur de tension pour permettre d'utiliser les composants traditionnels, rend possible de mélanger les fonctions de charge et de détection. Avec un régulateur de tension cela serait impossible de réunir les broches.

## Revendications

1. Couvert intelligent pour guider l'utilisateur dans sa vitesse d'alimentation réalisé par l'assemblage d'une tête de couvert (1) d'un manche de couvert (2) assemblé mécaniquement avec un boitier de protection (30) d'une carte électronique (10), la carte électronique (10) comprend deux bornes d'alimentation (B+, B-) et une plage de contact Moins (12) qui est reliée directement à la borne d'alimentation Moins (B-) du circuit, une plage de contact Plus (11) reliée à travers le circuit de cœur (17) à la borne (B+), des ressorts de contacts (21, 22) font respectivement contact entre la tête de couvert (1) et le plage de contact Plus (11) et entre le manche de couvert (2) et la plage de contact Moins (12) **caractérisé en ce que** le boitier de protection (30) est étanchéifié par une fermeture inviolable, l'alimentation est logée dans le boitier de protection (30) et est une super capacité (20) rechargeable, le couvert est rechargeable dans un étui (50) en positionnant au moyen d'un circuit de calage (58) en contact la tête de couvert à la borne Plus d'une alimentation externe (53) et le manche de couvert à la borne Moins de cette alimentation externe (53).

2. Couvert intelligent pour guider l'utilisateur dans sa vitesse d'alimentation selon la revendication 1 **caractérisé en ce que** le circuit de cœur (17) de la carte électronique (10) comprend un circuit d'avertissement (102) avec des sorties d'avertissement (15), vibreur (15a), lumière LED (15b), les sorties d'avertissement étant pilotées par une sortie sur la puce d'une puce de calcul (87), un circuit de détection (101) qui détecte les mises en bouche de la tête de couvert au moyen d'un circuit de division de tension sur alimentation non régulée, par couplage de résistance de tirage (83), de résistance de masse (82) et de transistor inversé (81) qui est branché sur une entrée de la puce de calcul (87), un circuit de charge (100) qui au moyen d'une diode au seuil inférieur à la tension de charge court-circuite astucieusement le circuit de détection (101), au cas où le circuit de charge soit alimenté par la source d'énergie externe (53).

## Patentansprüche

1. - Intelligentes Besteck zur Steuerung der Essgeschwindigkeit des Benutzers, hergestellt durch die Montage eines Besteckkopfes (1) mit einem Besteckgriff (2), der mechanisch mit einem Schutzgehäuse (30) einer elektronischen Karte (10) zusammengebaut ist, wobei die elektronische Karte (10) zwei Stromversorgungsanschlüsse (B+, B-) und ein Minuskontaktfeld (12) aufweist, das direkt mit dem Minusstromversorgungsanschluss (B-) der Schaltung verbunden ist, wobei ein Pluskontaktfeld (11) über die Kernschaltung (17) mit dem Anschluss (B+) verbunden ist, wobei Kontaktfedern (21, 22) jeweils den Kontakt zwischen dem Besteckkopf (1) und dem Pluskontaktfeld (11) sowie zwischen dem Besteckgriff (2) und dem Minuskontaktfeld (12) herstellen, **dadurch gekennzeichnet, dass** das Schutzgehäuse (30) durch einen Sicherheitsverschluss versiegelt ist, die Stromversorgung im Schutzgehäuse (30) untergebracht ist und eine wiederaufladbare Superkapazität (20) ist, Das Besteck ist in einem Etui (50) wiederaufladbar, indem mittels einer Zeitschaltung (58) der Besteckkopf mit dem Pluspol einer externen Stromversorgung (53) und der Besteckgriff mit dem Minuspol dieser externen Stromversorgung (53) in Kontakt gebracht wird.

2. - Intelligentes Besteck zur Steuerung der Essgeschwindigkeit des Benutzers nach Anspruch 1, **dadurch gekennzeichnet, dass** die Kernschaltung (17) der elektronischen Karte (10) eine Warnschaltung (102) mit Warnausgängen (15), Summer (15a) und LED-Licht (15b) umfasst, wobei die Warnausgänge von einem Ausgang auf dem Chip eines Rechenchips (87) angesteuert werden, eine Erkennungsschaltung (101), die das Einführen des Besteckkopfes mittels einer Spannungsteilerschaltung bei ungeregelter Stromversorgung erkennt, durch Kopplung eines Pull-Up-Widerstands (83), eines Erdungswiderstands (82) und eines invertierten Transistors (81), der mit einem Eingang des Rechenchips (87) verbunden ist, und eine Ladeschaltung (100), die mittels einer Diode bei einem Schwellenwert, der niedriger ist als die Ladespannung, die Erkennungsschaltung (101) geschickt kurzschließt, falls die Ladeschaltung von der externen Energiequelle (53) gespeist wird.

## Claims

1. - Intelligent cutlery for guiding the user in its feeding speed made by the assembly of a cutlery head (1) of a cutlery handle (2) mechanically assembled with a protective housing (30) of an electronic card (10), the electronic card (10) comprises two power supply terminals (B +, B-) and a Minus contact pad (12) which is directly connected to the Minus power supply terminal (B-) of the circuit, a Plus contact pad (11) connected through the core circuit (17) to the terminal (B +), contact springs (21, 22) respectively make contact between the cutlery head (1) and the Plus contact pad (11) and between the cutlery handle (2) and the Minus contact pad (12) **characterized in that** the protective housing (30) is sealed by a tamper-evident closure, the power supply is housed in the protective housing (30) and is a rechargeable super capacity (20), the cutlery is rechargeable in a case (50) by positioning by means of a timing circuit (58) in contact the cutlery head with the Plus terminal of an external power supply (53) and the cutlery handle with the Minus terminal of this external power supply (53).

2. - Intelligent cutlery for guiding the user in his feeding speed according to claim 1 **characterized in that** the core circuit (17) of the electronic card (10) comprises a warning circuit (102) with warning outputs (15), buzzer (15a), LED light (15b), the warning outputs being driven by an output on the chip of a computing chip (87), a detection circuit (101) which detects the mouthing of the cutlery head by means of a voltage division circuit on unregulated power supply, by coupling of pull-up resistor (83), ground resistor (82) and inverted transistor (81) which is connected to an input of the computing chip (87), a charging circuit (100) which by means of a diode at the threshold lower than the charging voltage cleverly short-circuits the detection circuit (101), in case the charging circuit is powered by the external energy source (53).
